# EUROPEAN PATENT SPECIFICATION

(11) **EP 2 942 004 B1**
(45) Date of publication and mention of the grant of the patent: **23.09.2020**
(21) Application number: 15001386.0
(22) Date of filing: 08.05.2015
(51) Int. Cl.: A61B 3/08, A61B 3/15

(54) **POSITIONING OF AUTOSTEREOSCOPIC TABLET COMPUTER**
POSITIONIERUNG EINES AUTOSTEREOSKOPISCHEN TABLET-COMPUTERS
POSITIONNEMENT D'UNE TABLETTE NUMÉRIQUE AUTOSTÉRÉOSCOPIQUE

(30) Priority: 09.05.2014 IT TO20140370
(43) Date of publication of application: 11.11.2015
(73) Proprietor: D.M.D. Computers S.r.l., 10090 Villarbasse (TO) (IT)
(72) Inventor: De Micheli, Spiridione, 10090 Villarbasse (TO) (IT)
(74) Representative: Aprà, Mario

(56) References cited:
- EP-A1- 1 444 945
- US-A1- 2011 025 976
- US-A1- 2013 155 376

## Description

The present invention relates to a process for determining the correct positioning of an autostereoscopic tablet computer used to perform fixation disparity measurements in a subject through haploscopic observation of targets or stimuli.

The present invention also relates to an apparatus for the implementation of the aforesaid method.

At the state of the art, there are known different techniques for performing fixation disparity measurements in a subject through haploscopic observation of targets.

US 2013/155376 discloses an alignment of a tablet for visual field testing, wherein the tablet displays a vertical line overlay and an image of the face of the subject, wherein the subject is provided with a marker which is to be aligned with the vertical line overlay.

More in particular, said measurements are performed by presenting the subject being examined with stimuli perceived by one eye and other stimuli perceived by the other eye, wherein, among other things, there is evaluated the perception of the lack of alignment of indices on the monitor of an autostereoscopic tablet computer, provided with frontal video camera means for shooting images and with image reproduction means for reproducing the images shot on a monitor of said autostereoscopic tablet computer (preferably in order improve implementation of said method said monitor has at least 1920 x 1200 pixels with a resolution of at least 100 pixels per mm²). The need for increasingly sophisticated diagnostics of the bulbus oculi of a subject, obtained by measuring the characteristic parameters linked to the sensorial process of visually capturing scenes external to the same subject, is directly linked to the need for increasingly greater precision in determining effective or suspected visual impairment.

On the other hand, due to the growing availability, at the state of the art, of increasingly high performance lenses (take, for example, "progressive lenses") to correct a specific visual impairment, there is the inevitable need for methods and apparatus for performing tests that allow reliable and highly precise data concerning the existing or suspected visual impairment of the subject to be obtained.

As mentioned, it is known to perform said measurements using tests and related apparatus, which allow the presence and extent of any fixation disparities to be identified.

By way of example, there is known the performance of measurement of the visual abilities of a subject by presenting stimuli for both binocular and monocular vision at distances comprised in a range between two and six meters (the "distance vision test").

Currently, the aforesaid type of visual ability measurement is combined with further techniques for the same measurement, but performed by presenting the subject with stimuli for both binocular and monocular vision at a distance between thirty and sixty centimeters (the "near vision test") and preferably forty centimeters.

As mentioned, in order to perform the aforesaid known measurement techniques, there is advantageously used a 10" autostereoscopic tablet computer, capable of presenting the subject being examined with some stimuli perceived by one eye, for example the right eye, and other stimuli only by the other eye, the left eye, the monitor of the same autostereoscopic tablet having at least 1920 x 1200 pixels and a resolution of at least 100 pixels per mm².

In order to obtain reliable results of the aforesaid evaluation and measurement operations, correct positioning of the autostereoscopic tablet computer by the subject being examined is a fundamental requirement; likewise, it is also important for the same autostereoscopic tablet computer to be maintained in the same correct position during the same measurement.

In this direction, one of the greatest problems encountered while performing said fixation disparity measurement operations by a subject, lies in the prejudice, in terms of poor precision and low reliability of the results obtained by the same measurement, due to incorrect positioning of the autostereoscopic tablet computer during the measurement.

In fact, it was noted in several cases that finding the correct positioning of the autostereoscopic tablet computer, and maintaining this positioning during the measurement, is often difficult and awkward for the subject being examined. This also becomes particularly evident when taking into due consideration the fact that the person being examined can be any subject, and as such not necessarily in possession of particular and specific technical knowledge.

On the other hand, said known methods do not allow the operator performing the measurement to be able to provide prompt and targeted instructions to the subject on whom the measurement is being performed concerning the correct positioning of the autostereoscopic tablet computer, or to check that correct positioning of the autostereoscopic tablet computer is maintained during the measurement.

An attempt to solve the aforesaid problems of the prior art was provided by means of a positioning process that provides for presenting, on the monitor of a tablet computer as said, two circular areas, one positioned on the right of the monitor of the tablet and the other on the left of the same monitor, in which the subject being examined is presented with uniform images, with shades of grey that vary according to the position with respect to the optimal position; in this case, the optimal positioning for measurement, which is difficult to achieve in operating condition, is the one in which the shades of grey in the two circular areas are perceived to be the same.

However, it is evident that a similar positioning process is by no means suitable to solve the aforesaid problems of the prior art, firstly making it very difficult for the subject, especially if without specific skills, to identify the exact correspondence of the shades of grey of said two circular areas, in order to determine the correct positioning of the autostereoscopic tablet computer.

Moreover, in order to check the exact correspondence of the shades of grey in the two circular areas, the subject on whom the measurement is being performed must continuously shift his/her fixation between the central area in which the stimulus is present, the left circular area and the right circular area, in which the areas of reference are provided, and therefore has to perform the test in conditions that are somewhat uncomfortable and anything but natural.

On the other hand, said known positioning process does not allow the operator performing the measurement to provide any kind of assistance and/or control, either in relation to the correct positioning of the autostereoscopic tablet computer, or in regard to subsequently maintaining the same in the correct position to perform the measurement.

Starting from the notion of these drawbacks, the present invention intends to provide a remedy.

An object of the present invention is to provide a process for determining the correct positioning of an autostereoscopic tablet computer used to perform fixation disparity measurements in a subject through haploscopic observation of targets, which allows the subject on whom the measurement is being performed to obtain, in complete ease, the correct positioning of the autostereoscopic tablet computer, and to maintain this positioning during the same measurement in a simple way, consequently reducing the time required to perform the test and considerably improving the reliability of the results of the same measurement.

Moreover, an object of the present invention is to provide a process as indicated which allows the operator performing the measurement to provide prompt and targeted instructions to the subject on whom the measurement is being performed in relation to correctness of the positioning of the autostereoscopic tablet computer, and to check that the positioning of said autostereoscopic tablet computer is correctly maintained during the same measurement.

On the other hand, an object of the present invention is to provide a process as said, which makes it easy to achieve the correct positioning of the autostereoscopic tablet computer, and subsequently to maintain said correct positioning during the measurement, also for subjects without particular technical knowledge.

Another object of the present invention is to provide an apparatus for the implementation of said process as mentioned. In view of these objects, the present invention provides for a process for determining the correct positioning of an autostereoscopic tablet computer used to perform fixation disparity measurements in a subject through haploscopic observation of targets, the essential characteristic of which forms the subject matter of claim 1 the present invention also provides for an apparatus for the aforesaid implementation, the essential characteristic of which forms the subject matter of claim 9. Further advantageous characteristics are described in the dependent claims.

The aforesaid claims are intended as fully incorporated herein.

The present invention will become more apparent from the following detailed description, with reference to the drawing attached hereto, which is purely exemplary and therefore nonlimiting, in which:

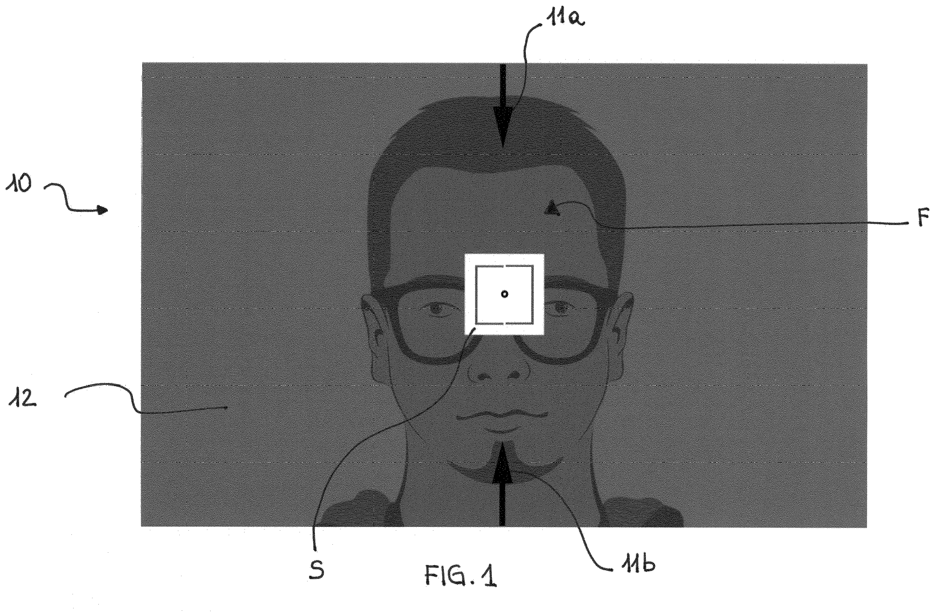

- Fig. 1 is a photographic reproduction of an autostereoscopic tablet computer viewed from the side of the monitor, the correct positioning of which for the purposes of performing the measurement is given by the fact that the ends of two indices are respectively positioned so that the upper index is positioned on the central and upper part of the forehead of the subject being examined and that the proximal end of the lower index is positioned on the tip of the nose of the same subject.

In the following, there is provided the description of an exemplary embodiment of the process for determining the correct positioning of an autostereoscopic tablet computer used to perform fixation disparity measurements in a subject through haploscopic observation of targets, according to the present invention.

According to what is known in the state of the art, the performance of fixation disparity measurements in a subject through haploscopic observation of targets essentially takes place according to the following steps of:
- performing a test by presenting the subject being examined, on said autostereoscopic tablet computer 10 with stimuli S perceived by one eye and other stimuli S perceived by the other eye,
- evaluating the perception of the lack of alignment of indices 11a, 11b or of the correct merging of different images, presented on the monitor 12 of said autostereoscopic tablet computer 10, provided with frontal video camera means for shooting images and with image reproduction means for reproducing the images shot on said monitor 12, having at least 1920 x 1200 pixels with a resolution of at least 100 pixels mm².

For the purposes of the aforesaid measurement, said autostereoscopic tablet computer 10, on which there is installed dedicated software, must be positioned with respect to the subject being examined, with said monitor 12 and said frontal video camera means facing the same subject, complying with the requirements of correct distance and inclination between the face of the subject being examined and the monitor 12 of said autostereoscopic tablet computer.

If the aforesaid requirements of correct distance and inclination between the face of the subject being examined and monitor 12 of said autostereoscopic tablet computer 10 are not sufficiently satisfied, there is an inevitable risk in terms of poor reliability of the results of the same measurement.

According to the present invention, said process for determining the correct positioning of an autostereoscopic tablet computer 10 used to perform fixation disparity measurements in a subject through haploscopic observation of targets, comprises the steps consisting of:
- providing a static image, on said monitor 12 of the autostereoscopic tablet computer 10, of two indices 11a, lib, one upper 11a and the other lower 11b, having respective proximal ends and mutually aligned according to a vertical transverse median plane of the same monitor 12 containing the optical axis of said video camera means;
- providing an image, on said monitor of the autostereoscopic tablet computer 10, of a stimulus S characteristic of the test;
- at the same time shooting an image F of the face of the subject being examined, by means of said frontal video camera means of the autostereoscopic tablet computer 10 and displaying the same image on said same monitor 12, in the background with respect to said image of the stimulus S characteristic of the test and presented on the same monitor 12 with sufficient contrast to perceive said image F in the background.

According to the invention, said autostereoscopic tablet computer 10, to satisfy said requirements of correct distance and inclination, is oriented and arranged, positioning said proximal ends of said indices 11a, 11b, one just above the stimulus S and the other just below the stimulus S, as shown in Fig. 1.

In this way, the subject being examined is placed in the conditions to determine, in complete ease, the correct positioning of said autostereoscopic tablet computer 10 for the performance of said fixation disparity measurements.

Advantageously, said autostereoscopic tablet computer 10 can be held by the hands of the subject being examined and is oriented and arranged, positioning said proximal ends of said indices 11a, 11b, the one just above the stimulus S and the other just below the stimulus S, so that the upper index 11a is positioned on the central and upper part of the forehead of the subject being examined and that said proximal end of the lower index 11b is positioned on the tip of the nose of the same subject.

In this way, the subject being tested performs the test in a totally natural way, maintaining a position more or less similar to the position maintained when reading a book while seated.

The naturalness of the position of the subject during performance of the measurement greatly facilitates maintaining the correct positioning of the tablet computer 10 during the measurement, and at the same time reduces the measurement time, with all the consequences in terms of reliability of the related results.

Alternatively, said autostereoscopic tablet computer 10 can be supported stably by means of a swivelling support (not shown); in this case, said swivelling support carrying said autostereoscopic tablet computer 10 is oriented by the hands of the subject being examined and arranged, positioning said proximal ends of said indices 11a, 11b, the one just above the stimulus S and the other just below the stimulus S, so that the upper index 11a is positioned on the central and upper part of the forehead of the subject being examined and that said proximal end of the lower index 11b is positioned on the tip of the nose of the same subject.

On the other hand, said swivelling support carrying said autostereoscopic tablet computer 10 can alternatively be oriented by the hands of an operator other than the subject being examined (for example, the operator in charge of performing the measurement) and thus correctly arranged, as indicated above.

In this way, once correct orientation of the autostereoscopic tablet computer 10 has been obtained, maintaining this orientation during the measurement is particularly easy and immediate, given that it is positioned on said support that cooperates to maintain the same in the desired position.

In order to further facilitate the correct positioning of the autostereoscopic tablet computer 10 by the subject on whom the measurement is being performed, said tablet computer 10 can be associated with a further (not shown) tablet computer (not autostereoscopic), on which there is installed dedicated software, which is operatively connected to said autostereoscopic tablet computer 10 by means of a signal transmission network, and on the monitor of which there are reproduced, in real time with respect to said autostereoscopic tablet computer 10:
- said static image, reproduced on said monitor of the autostereoscopic tablet computer 10, of two indices 11a, 11b, one upper 11a and the other lower 11b, having respective proximal ends and mutually aligned according to a vertical transverse median plane of the same monitor 12 containing the optical axis of said video camera means;
- said image, reproduced on said monitor of the autostereoscopic tablet computer, of a stimulus S characteristic of the test;
- said image F of the face of the subject being examined, shot by means of said frontal video camera means of the autostereoscopic tablet computer 10 and displayed on the monitor 12 thereof, in the background with respect to said image of the stimulus S characteristic of the test and presented on the monitor 12 of said autostereoscopic tablet computer 10 with just sufficient contrast to perceive said image F in the background.

Said further tablet computer (not shown), advantageously assigned to the operator who performs the same measurement, allows this latter to display on the related monitor the same images simultaneously displayed to the subject being examined, so as to be able to provide the same with useful instructions in order to facilitate the correct positioning of his/her tablet computer 10, and to check, during the measurement, that the aforesaid correct positioning is maintained.

Advantageously, the process according to the invention comprises a step of selectively controlling the display, on the monitor 12 of said autostereoscopic tablet computer 10, of said static image of two indices 11a, 11b, of said at least one image of a stimulus S and/or of said image F of the face of the subject being examined, through remote control means of said display provided in said further tablet computer, so that one, none or a plurality of said same images are visible on the monitor 12 of said autostereoscopic tablet computer. According to the preference of the subject being examined, said image F displayed in the background with respect to said image of the stimulus S characteristic of the test, on the monitor 12 of said autostereoscopic tablet computer 10, can be controlled by means of timed means that cause the disappearance thereof after a predetermined interval of time. Moreover, this does not exclude said image F from continuing to be present on said further tablet computer (if present) assigned to the operator, so that the - latter is in a position to be able to check, in an easy and immediate way, that the correct positioning of the autostereoscopic tablet computer 10 is maintained during the whole performance of the test.

Advantageously, said image of said stimulus S is reproduced on said monitor 12 of said autostereoscopic tablet computer 10 in a box outside which the remaining part of said monitor 12 is grey (as shown in Fig. 1) or filled with characters with the aim of creating confusion.

It is understood that different modes of reproduction of said image of said stimulus, provided that they are suitable for the purpose, also fall within the scope of the present invention.

Apparatus for determining the correct positioning of an autostereoscopic tablet computer used to perform fixation disparity measurements in a subject through haploscopic observation of targets.

In the following, there is provided the description of an exemplary embodiment of an apparatus for determining the correct positioning of an autostereoscopic tablet computer 10 used to perform fixation disparity measurements in a subject through haploscopic observation of targets, by means of the process according to the present invention.

Said apparatus comprises:
- a static image, on the monitor 12 of said autostereoscopic tablet computer 10, of two indices 11a, 11b, one upper 11a and the other lower 11b, having respective proximal ends and mutually aligned according to a vertical transverse median plane of the same monitor 12 containing the optical axis of said video camera means;
- an image, on said monitor 12 of the autostereoscopic tablet computer 10, of a stimulus S characteristic of the test;
- an image F of the face of the subject being examined, shot by means of said frontal video camera means of the autostereoscopic tablet computer 10 and displayed on said same monitor 12, in the background with respect to said image of the stimulus S characteristic of the test and presented on the same monitor 12 with just sufficient contrast to perceive said image F in the background.

In order to satisfy said requirements of correct distance and inclination, the autostereoscopic tablet computer 10 is oriented and arranged, positioning said proximal ends of said indices 11a, 11b, the one just above the stimulus S and the other just below the stimulus S, so that the upper index 11a is positioned on the central and upper part of the forehead of the subject being examined and that said proximal end of the lower index 11b is positioned on the tip of the nose of the same subject.

Advantageously, said image of said stimulus S occupies a box of said monitor 12 of said autostereoscopic tablet computer 10 outside which the remaining part of said monitor 12 is grey or filled with characters with the aim of creating confusion.

It is understood that different modes of reproduction of said image of said stimulus, provided that they are suitable for the purpose, fall within the scope of the present invention. According to the embodiment of the invention illustrated, said two indices 11a, 11b, one upper and the other lower are pointed, with points facing each other, and mutually aligned according to a vertical transverse median plane of the monitor 12 of the tablet computer 10 containing the optical axis of said video camera means.

Evidently, different structures of said indices (not shown), provided that they are suitable for the purpose, are also possible according to the invention.

According to preference, said autostereoscopic tablet computer 10 can be supported stably by means of a swivelling support (not shown); alternatively, the same can be supported manually by the subject on whom the measurement is being performed, essentially in the manner of a book.

Advantageously, said autostereoscopic tablet computer 10, on which there is installed dedicated software, is operatively connected with respect to a further tablet computer (not shown), by means of a signal transmission network, so that on the monitor of said further tablet computer there are reproduced, in real time with respect to said autostereoscopic tablet computer:
- said static image, reproduced on said monitor 12 of the autostereoscopic tablet computer 10, of two indices 11a, 11b, one upper 11a and the other lower 11b, having respective proximal ends and mutually aligned according to a vertical transverse median plane of the same monitor 12 containing the optical axis of said video camera means;
- said image, reproduced on said monitor 12 of the autostereoscopic tablet computer 10, of a stimulus S characteristic of the test;
- said image F of the face of the subject being examined, shot by means of said frontal video camera means of the autostereoscopic tablet computer 10 and displayed on the monitor 12 thereof, in the background with respect to said image of the stimulus S characteristic of the test and presented on the monitor 12 of said autostereoscopic tablet computer 10 with just sufficient contrast to perceive said image F in the background.

Said further tablet computer (not shown), advantageously assigned to the operator who performs the same measurement or evaluation, allows this latter to display on the related monitor the same images simultaneously displayed to the subject being examined, so as to be able to provide this latter with useful instructions in order to facilitate the correct positioning of his/her tablet computer 10, and to check, during the measurement, that the aforesaid correct positioning is maintained. Advantageously, said apparatus comprises, according to the invention, remote control means, provided in said further tablet computer, of the display, on the monitor 12 of said autostereoscopic tablet computer 10, of said static image of two indices 11a, 11b, of said at least one image of a stimulus S and/or of said image F of the face of the subject being examined, which selectively allow one, none or a plurality of said same images to be displayed on the monitor 12 of said autostereoscopic tablet computer 10.

As is apparent from the above, said process for determining the correct positioning of an autostereoscopic tablet computer used for performing fixation disparity measurements in a subject through haploscopic observation of targets, according to the present invention, allows the subject on whom the same measurement is being performed to obtain, in complete ease, the correct positioning of the autostereoscopic tablet computer, and to maintain this positioning during the same measurement in a simple way, with all the consequences in terms of considerably improving the reliability of the same measurement.

Moreover, said process as indicated allows the operator performing the measurement to provide prompt and targeted instructions to the subject on whom the measurement is being performed concerning the correct positioning of the autostereoscopic tablet computer, and to check that correct positioning of the autostereoscopic tablet computer is maintained during the measurement.

On the other hand, said process as mentioned, makes it easy to attain the correct positioning of the autostereoscopic tablet computer, and subsequently to maintain said correct positioning during performance of the measurement, also for subjects without particular technical knowledge.

As is apparent, the present invention allows the objects described in the introduction to be achieved in a simple and advantageous manner.

## Claims

1. Process for determining the correct positioning of an autostereoscopic tablet computer (10) used to perform fixation disparity measurements in a subject through haploscopic observation of stimuli,
said autostereoscopic tablet computer comprising:
- frontal video camera means for capturing images of the face of the subject, said frontal video camera means defining an optical axis,
- a monitor (12) having at least 1920 x 1200 pixels with a resolution of at least 100 pixels per mm², the monitor defining a vertical transverse median plane containing said optical axis,
- image reproduction means for reproducing images captured by said video camera means on said monitor, and
- dedicated software;
wherein a fixation disparity measurement is performed by presenting to the subject being examined, on said autostereoscopic tablet computer (10), stimuli (S) perceived by one eye and other stimuli (S) perceived by the other eye,
wherein said measurement comprises the steps consisting of:
- providing a static image, on said monitor (12), of an upper index (11a) and a lower index (11b), aligned according to said vertical transverse median plane ;
- providing at least one image, on said monitor (12), of a stimulus (S) characteristic of the measurement;
- when said at least one image of the stimulus (S) is provided on said monitor (12), capturing an image (F) of the face of the subject being examined, by means of said frontal video camera means and displaying this image on the monitor (12), in the background with respect to said image of the stimulus (S) with sufficient contrast to perceive said image (F) of the face in the background,
wherein, for achieving correct positioning of the autostereoscopic tablet computer (10), said autostereoscopic tablet computer (10) is oriented and arranged by positioning said indices (11a, 11b) the one just above the stimulus (S) and the other just below the stimulus (S), so that the upper index (11a) is positioned on the central and upper part of the forehead of the subject being examined and the lower index (11b) is positioned on the tip of the nose of the same subject.

2. Process according to claim 1, **characterized in that** said autostereoscopic tablet computer (10) is held by the hands of the subject being examined and is oriented and arranged, positioning of said indices (11a, 11b), the one just above the stimulus (S) and the other just below the stimulus (S), so that the upper index (11a) is positioned on the central and upper part of the forehead of the subject being examined and the lower index (11b) is positioned on the tip of the nose of the same subject.

3. Process according to claim 1, **characterized in that** said autostereoscopic tablet computer (10) is supported stably by means of a swivelling support, and **in that** said swivelling support carrying said autostereoscopic tablet computer (10) is oriented by the hands of the subject being examined and arranged, positioning said indices (11a, 11b), the one just above the stimulus (S) and the other just below the stimulus (S), so that the upper index (11a) is positioned on the central and upper part of the forehead of the subject being examined and the lower index (11b) is positioned on the tip of the nose of the same subject.

4. Process according to claim 1, **characterized in that** said autostereoscopic tablet computer (10) is supported stably by means of a swivelling support, and **in that** said swivelling support carrying said autostereoscopic tablet computer is oriented by the hands of an operator other than the subject being examined and arranged, positioning said indices (11a, 11b), the one just above the stimulus (S) and the other just below the stimulus (S), so that the upper index (11a) is positioned on the central and upper part of the forehead of the subject being examined and the lower index (11b) is positioned on the tip of the nose of the same subject.

5. Process according to one or more of the preceding claims, **characterized in that** said autostereoscopic tablet computer (10) is associated with a further tablet computer, on which there is installed dedicated software, which is operatively connected to said autostereoscopic tablet computer (10) by means of a signal transmission network, and on the monitor of which there are reproduced, in real time with respect to said autostereoscopic tablet computer (10):
- said static image, reproduced on said monitor of the autostereoscopic tablet computer (10), of two indices (11a, 11b), one upper (11a) and the other lower (11b), mutually aligned according to a vertical transverse median plane of the same monitor (12) containing the optical axis of said video camera means;
- said at least one image, reproduced on said monitor of the autostereoscopic tablet computer, of a stimulus (S) characteristic of the measurement;
- said image (F) of the face of the subject being examined, shot by means of said frontal video camera means of the autostereoscopic tablet computer (10) and displayed on the monitor (12) thereof, in the background with respect to said image of the stimulus (S) characteristic of the measurement and presented on the monitor (12) of said autostereoscopic tablet computer (10) with just sufficient contrast to perceive said image (F) in the background.

6. Process according to claim 5, **characterized in that** it comprises a step of selectively controlling the display, on the monitor (12) of said autostereoscopic tablet computer (10), of said static image of two indices (11a, 11b), of said at least one image of a stimulus (S) and/or of said image (F) of the face of the subject being examined, through remote control means of said display provided in said further tablet computer, so that one, none or a plurality of said same images are visible on the monitor (12) of said autostereoscopic tablet computer (10).

7. Process according to one or more of the preceding claims, **characterized in that** said image (F) displayed in the background with respect to said image of the stimulus (S) characteristic of the measurement, on the monitor (12) of said autostereoscopic tablet computer (10), is controlled by means of timed means that cause the disappearance thereof after a predetermined interval of time.

8. Process according to one or more of the preceding claims, **characterized in that** said at least one image of said stimulus (S) is reproduced on said monitor (12) of said autostereoscopic tablet computer (10) in a box outside which the remaining part of said monitor (12) is grey or filled with characters with the aim of creating confusion.

9. Autostereoscopic tablet computer (10) configured to perform fixation disparity measurements in a subject through haploscopic observation of stimuli, according to one or more of the preceding claims, wherein said tablet computer comprises:
- frontal video camera means for capturing images of the face of the subject, said frontal video camera means defining an optical axis,
- a monitor (12) having at least 1920 x 1200 pixels with a resolution of at least 100 pixels per mm², the monitor defining a vertical transverse median plane containing said optical axis,
- image reproduction means for reproducing images captured by said video camera means on said monitor, and
- dedicated software;
- said monitor being configured to display : a static image of an upper index (11a) and a lower index (11b), mutually aligned according to said vertical transverse median plane ;
- at least one image of a stimulus (S) characteristic of the measurement;
- an image (F) of the face of the subject, captured by said frontal video camera means in the background with respect to said image of the stimulus (S) with just sufficient contrast to perceive said image (F) of the face in the background,
so that, for achieving correct positioning of the autostereoscopic tablet computer (10), said autostereoscopic tablet computer (10) can be oriented and arranged by positioning said indices (11a, 11b) the one just above the stimulus (S) and the other just below the stimulus (S), so that the upper index (11a) is positioned on the central and upper part of the forehead of the subject being examined and that the lower index (11b) is positioned on the tip of the nose of the same subject.

10. Autostereoscopic tablet computer according to claim 9, **characterized in that** said at least one image of said stimulus (S) occupies a box of said monitor of said autostereoscopic tablet computer (10) outside which the remaining part of said monitor (12) is grey or filled with characters with the aim of creating confusion.

11. Autostereoscopic tablet computer according to claim 9 and/or 10, **characterized in that** said two indices (11a, 11b), one upper (11a) and the other lower (11b) are pointed, with points facing each other, and mutually aligned according to said vertical transverse median plane.

12. Autostereoscopic tablet computer according to claim 9, **characterized in that** said autostereoscopic tablet computer (10) is supported stably by means of a swivelling support.

13. Autostereoscopic tablet computer according to one or more of claims 9 to 12, **characterized in that** said autostereoscopic tablet computer (10), on which dedicated software is installed, is operatively connected to a further tablet computer, by means of a signal transmission network, and **in that** on the monitor of said further table computer there are reproduced, in real time with respect to said autostereoscopic tablet computer:
- said static image, reproduced on said monitor (12) of the autostereoscopic tablet computer (10), of two indices (11a, 11b), one upper (11a) and the other lower (11b), mutually aligned according to a vertical transverse median plane of the same monitor (12) containing the optical axis of said video camera means;
- said at least one image, reproduced on said monitor (12) of the autostereoscopic tablet computer (10), of a stimulus (S) characteristic of the measurement;
- said image (F) of the face of the subject being examined, captured by means of said frontal video camera means of the autostereoscopic tablet computer (10) and displayed on the monitor (12) thereof, in the background with respect to said image of the stimulus (S) characteristic of the measurement and presented on the monitor (12) of said autostereoscopic tablet computer (10) with just sufficient contrast to perceive said image (F) in the background.

14. Autostereoscopic tablet computer according to claim 13, **characterized in that** it comprises remote control means, provided in said further tablet computer, of the display, on the monitor (12) of said autostereoscopic tablet computer (10) of said static image of two indices (11a, 11b), of said at least one image of a stimulus (S) and/or of said image (F) of the face of the subject being examined, which allow one, none or a plurality of said same images to be made visible on the monitor (12) of said autostereoscopic tablet computer (10).

## Patentansprüche

1. Verfahren zur Bestimmung der korrekten Positionierung eines autostereoskopischen Tabletcomputers (10), der zur Durchführung von Messungen der Fixationsdisparität bei einem Subjekt durch haploskopische Beobachtung von Stimuli benutzt wird,
wobei der besagte autostereoskopische Tabletcomputer Folgendes umfasst:
- eine frontale Videokameravorrichtung zum Erfassen von Bildern des Gesichts des Subjekts, wobei die besagte frontale Videokameravorrichtung eine optische Achse definiert,
- einen Monitor (12) mit mindestens 1920 x 1200 Pixeln mit einer Auflösung von mindestens 100 Pixeln pro mm², wobei der Monitor eine vertikale transversale Mittelebene definiert, die die besagte optische Achse enthält,
- Bildwiedergabemittel zur Wiedergabe von Bildern, die von den besagten Videokameravorrichtung auf dem besagten Monitor erfasst wurden, und
- dedizierte Software;
wobei eine Messung der Fixationsdisparität durchgeführt wird, indem dem zu untersuchenden Subjekt auf dem besagten autostereoskopischen Tabletcomputer (10) Stimuli (S), die von einem Auge wahrgenommen werden, und andere Stimuli (S), die von dem anderen Auge wahrgenommen werden, präsentiert werden, wobei die besagte Messung folgende Schritte umfasst, bestehend aus:
- Bereitstellen eines statischen Bildes auf dem besagten Monitor (12) eines oberen Index (11a) und eines unteren Index (11b), die gemäß der besagten vertikalen transversalen Mittelebene ausgerichtet sind
- Bereitstellen mindestens eines Bildes auf dem besagten Monitor (12) eines für die Messung charakteristischen Stimulus (S);
- wenn das besagte mindestens eine Bild des Stimulus (S) auf dem besagten Monitor (12) bereitgestellt wird, erfassend ein Bild (F) des Gesichts des zu untersuchenden Subjekts mit Hilfe der besagten frontalen Videokameravorrichtung und dieses Bild auf dem Monitor (12) im Hintergrund in Bezug auf das besagte Bild des Stimulus (S) mit ausreichendem Kontrast angezeigt wird, um das besagte Bild (F) des Gesichts im Hintergrund wahrzunehmen,
wobei, zum Erreichen einer korrekten Positionierung des autostereoskopischen Tabletcomputers (10) wird der besagte autostereoskopische Tabletcomputer (10) ausgerichtet und angeordnet, indem die besagten Indizes (11a, 11b), der eine unmittelbar über dem Stimulus (S) und der andere unmittelbar unter dem Stimulus (S), so positioniert werden, dass der obere Index (11a) auf dem zentralen und oberen Teil der Stirn des zu untersuchenden Subjekts und der untere Index (11b) auf der Nasenspitze desselben Subjekts positioniert wird.

2. Verfahren gemäß Anspruch 1, **dadurch gekennzeichnet, dass** der besagte autostereoskopische Tabletcomputer (10) von den Händen des zu untersuchenden Subjekts gehalten und ausgerichtet und angeordnet wird, indem die besagten Indizes (11a, 11b), der eine unmittelbar über dem Stimulus (S) und der andere unmittelbar unter dem Stimulus (S), so positioniert werden, dass der obere Index (11a) auf dem mittleren und oberen Teil der Stirn des zu untersuchenden Subjekts und der untere Index (11b) auf der Nasenspitze desselben Subjekts positioniert wird.

3. Verfahren gemäß Anspruch 1, **dadurch gekennzeichnet, dass** der besagte autostereoskopische Tabletcomputer (10) mittels einer schwenkbaren Stütze stabil gehalten wird, und dass die besagte schwenkbare Stütze, die den besagten Tabletcomputer (10) trägt, von den Händen des zu untersuchenden Subjekts ausgerichtet und angeordnet wird, indem die besagten Indizes (11a, 11b), der eine unmittelbar über dem Stimulus (S) und der andere unmittelbar unter dem Stimulus (S), so positioniert werden, dass der obere Index (11a) auf dem mittleren und oberen Teil der Stirn des zu untersuchenden Subjekts und der untere Index (11b) auf der Nasenspitze desselben Subjekts positioniert ist.

4. Verfahren gemäß Anspruch 1, **dadurch gekennzeichnet, dass** der besagte autostereoskopische Tabletcomputer (10) mittels einer schwenkbaren Stütze stabil gehalten wird und dass die besagte schwenkbare Stütze, die den besagten autostereoskopischen Tabletcomputer trägt, von den Händen eines anderen Bedieners als des zu untersuchenden Subjekts ausgerichtet und angeordnet wird, indem die besagten Indizes (11a, 11b), der eine unmittelbar über dem Stimulus (S) und der andere unmittelbar unter dem Stimulus (S), so positioniert werden, dass der obere Index (11a) auf dem mittleren und oberen Teil der Stirn des zu untersuchenden Subjekts und der untere Index (11b) auf der Nasenspitze desselben Subjekts positioniert ist.

5. Verfahren gemäß einem oder mehreren der vorhergehenden Ansprüche, **dadurch gekennzeichnet, dass** der besagte autostereoskopische Tabletcomputer (10) mit einem weiteren Tabletcomputer verbunden wird, auf dem eine dedizierte Software installiert ist, die mit dem besagten autostereoskopischen Tabletcomputer (10) mittels eines Signalübertragungsnetzwerks operativ verbunden ist und auf dessen Monitor in Echtzeit in Bezug auf den besagten autostereoskopischen Tabletcomputer (10) reproduziert wird:
- das besagte auf dem besagten Monitor des autostereoskopischen Tabletcomputers (10) reproduzierte statische Bild aus zwei Indizes (11a, 11b), einem oberen (11a) und einem unteren (11b), die gegenseitig gemäß einer vertikalen, transversalen Mittelebene desselben Monitors (12) ausgerichtet sind, enthält die optische Achse der besagten Videokameramittel;
- das besagte auf dem besagten Monitor des autostereoskopischen Tabletcomputers reproduzierte mindeste eine Bild eines für die Messung charakteristischen Stimulus (S);
- das besagte Bild (F) des Gesichts des zu untersuchenden Subjekts, aufgenommen mit Hilfe der besagten frontalen Videokameravorrichtung des autostereoskopischen Tabletcomputers (10) und angezeigt auf dessen Monitor (12), im Hintergrund in Bezug auf das besagte Bild des Stimulus (S), der für die Messung charakteristisch ist und auf dem Monitor (12) des besagten autostereoskopischen Tabletcomputers (10) mit gerade ausreichendem Kontrast präsentiert wird, um das besagte Bild (F) im Hintergrund wahrzunehmen.

6. Verfahren gemäß Anspruch 5, **dadurch gekennzeichnet, dass** es einen Schritt der selektiven Steuerung der Anzeige auf dem Monitor (12) des besagten autostereoskopischen Tabletcomputers (10), des besagten statischen Bildes der beiden Indizes (11a, 11b), des besagten mindestens einen Bildes eines Stimulus (S) und/oder des besagten Bildes (F) des Gesichts des zu untersuchenden Subjekts durch Fernsteuerungsmittel der besagten Anzeige, die in dem besagten weiteren Tabletcomputer bereitgestellt wird, umfasst, so dass eines, keines oder eine Vielzahl der besagten gleichen Bilder auf dem Monitor (12) des besagten autostereoskopischen Tabletcomputers (10) zu sehen sind.

7. Verfahren gemäß einem oder mehreren der vorhergehenden Ansprüche, **dadurch gekennzeichnet, dass** das besagte Bild (F), das im Hintergrund in Bezug auf das besagte Bild des für die Messung charakteristischen Stimulus (S) auf dem Monitor (12) des besagten autostereoskopischen Tabletcomputers (10) angezeigt wird, mittels zeitgesteuerter Mittel gesteuert wird, die sein Verschwinden nach einem vorherbestimmten Zeitintervall bewirken.

8. Verfahren gemäß einem oder mehreren der vorhergehenden Ansprüche, **dadurch gekennzeichnet, dass** das besagte mindestens eine Bild des besagten Stimulus (S) auf dem besagten Monitor (12) des besagten autostereoskopischen Tabletcomputers (10) in einem Kasten reproduziert wird, außerhalb dessen der verbleibende Teil des besagten Monitors (12) grau oder mit Zeichen gefüllt ist, mit dem Ziel, Verwirrung zu stiften.

9. Autostereoskopischer Tabletcomputer (10), konfiguriert, um Messungen der Fixationsdisparität bei einem Subjekt durch haploskopische Beobachtung von Stimuli durchzuführen, gemäß einem oder mehreren der vorhergehenden Ansprüche,
wobei der besagte Tabletcomputer Folgendes umfasst:
- eine frontale Videokameravorrichtung zum Aufnehmen von Bildern des Gesichts des Subjekts, wobei die besagte frontale Videokameravorrichtung eine optische Achse definiert,
- einen Monitor (12) mit mindestens 1920 x 1200 Pixeln mit einer Auflösung von mindestens 100 Pixeln pro mm², wobei der Monitor eine vertikale transversale Mittelebene definiert, die die besagte optische Achse enthält,
- Bildwiedergabemittel zur Wiedergabe von Bildern, die von der besagten Videokameravorrichtung auf dem besagten Monitor erfasst wurden, und
- dedizierte Software;
- wobei der besagte Monitor so konfiguriert ist, dass er ein statisches Bild eines oberen Index (11a) und eines unteren Index (11b) anzeigt, die entsprechend der besagten vertikalen transversalen Mittelebene aufeinander ausgerichtet sind;
- mindestens ein Bild eines für die Messung charakteristischen Stimulus (S);
- ein Bild (F) des Gesichts des Subjekts, das durch die besagte frontale Videokameravorrichtung im Hintergrund in Bezug auf das besagte Bild des Stimulus (S) mit gerade ausreichendem Kontrast erfasst wurde, um das besagte Bild (F) des Gesichts im Hintergrund wahrzunehmen, so dass zum Erreichen einer korrekten Positionierung des autostereoskopischen Tabletcomputers (10) der besagte autostereoskopische Tabletcomputer (10) ausgerichtet und angeordnet werden kann, indem die besagten Indizes (11a, 11b), der eine unmittelbar über dem Stimulus (S) und der andere unmittelbar unter dem Stimulus (S), so positioniert werden, dass der obere Index (11a) auf dem mittleren und oberen Teil der Stirn des zu untersuchenden Subjekts positioniert ist und dass der untere Index (11b) auf der Nasenspitze desselben Subjekts positioniert ist.

10. Autostereoskopischer Tabletcomputer gemäß Anspruch 9, **dadurch gekennzeichnet, dass** das besagte mindestens eine Bild des besagten Stimulus (S) einen Kasten des besagten Monitors des besagten autostereoskopischen Tabletcomputers (10) einnimmt, außerhalb dessen der verbleibende Teil des besagten Monitors (12) grau oder mit Zeichen gefüllt ist, mit dem Ziel, Verwirrung zu stiften.

11. Autostereoskopischer Tabletcomputer gemäß Anspruch 9 und/oder 10, **dadurch gekennzeichnet, dass** die besagten zwei Indizes (11a, 11b), ein oberer (11a) und ein unterer (11b) spitz zulaufen, wobei die Punkte einander gegenüberliegen und gemäß der besagten vertikalen transversalen Mittelebene aufeinander ausgerichtet sind.

12. Autostereoskopischer Tabletcomputer gemäß Anspruch 9, **dadurch gekennzeichnet, dass** der besagte autostereoskopische Tabletcomputer (10) mittels einer schwenkbaren Stütze stabil gehalten wird.

13. Autostereoskopischer Tabletcomputer gemäß einem oder mehreren der Ansprüche 9 bis 12, **dadurch gekennzeichnet, dass** der besagte autostereoskopische Tabletcomputer (10), auf dem eine dedizierte Software installiert ist, mit einem weiteren Tabletcomputer mittels eines Signalübertragungsnetzwerks operativ verbunden ist, und dass auf dem Monitor des besagten weiteren Tabletcomputers in Echtzeit in Bezug auf den besagten autostereoskopischen Tabletcomputer Folgendes reproduziert wird:
- das besagte auf dem besagten Monitor (12) des autostereoskopischen Tabletcomputers (10) reproduzierte statische Bild von zwei Indizes (11a, 11b), einem oberen (11a) und einem unteren (11b), die gemäß einer vertikalen transversalen Mittelebene desselben Monitors (12), die die optische Achse der besagten Videokameravorrichtung enthält, aufeinander ausgerichtet sind;
- das besagte mindestens eine auf dem besagten Monitor (12) des autostereoskopischen Tabletcomputers (10) reproduzierte Bild eines für die Messung charakteristischen Stimulus (S);
- das besagte Bild (F) des Gesichts des zu untersuchenden Subjekts, das mittels der besagten frontalen Videokameravorrichtung des autostereoskopischen Tabletcomputers (10) erfasst und auf dessen Monitor (12) im Hintergrund in Bezug auf das besagte Bild des Stimulus (S) angezeigt wird, der für die Messung charakteristisch ist und auf dem Monitor (12) des besagten autostereoskopischen Tabletcomputers (10) mit gerade ausreichendem Kontrast präsentiert wird, um das besagte Bild (F) im Hintergrund wahrzunehmen.

14. Autostereoskopischer Tabletcomputer gemäß Anspruch 13, **dadurch gekennzeichnet, dass** er umfasst in dem besagten weiteren Tabletcomputer bereitgestellte Fernsteuerungsmittel der Anzeige auf dem Monitor (12) des besagten auto stereoskopischen Tabletcomputers (10) des besagten statischen Bildes von zwei Indizes (11a, 11b), des besagten mindestens eines Bildes eines Stimulus (S) und/oder des besagten Bildes (F) des Gesichts des zu untersuchenden Subjekts, das es erlaubt, dass eines, keines oder eine Vielzahl der besagten gleichen Bilder auf dem Monitor (12) des besagten autostereoskopischen Tabletcomputers (10) zu sehen sind.

## Revendications

1. Procédé pour déterminer le positionnement correct d'une tablette informatique autostéréoscopique (10) utilisée pour effectuer des mesures de disparité de fixation chez un sujet par observation haploscopique de mires ou de stimuli, ladite tablette numérique autostéréoscopique comprenant :
- des moyens de caméra vidéo frontale pour capturer des images du visage du sujet, lesdits moyens de caméra vidéo frontale définissant un axe optique,
- un moniteur (12) ayant au moins 1920 x 1200 pixels avec une résolution d'au moins 100 pixels par mm², le moniteur définissant un plan médian transversal vertical contenant ledit axe optique,
- des moyens de reproduction d'images pour reproduire des images capturées par lesdits moyens de caméra vidéo sur ledit moniteur, et
- un logiciel dédié ;
où une mesure de disparité de fixation est effectuée en présentant au sujet examiné, sur ladite tablette numérique autostéréoscopique (10) des stimuli (S) perçus par un œil et d'autres stimuli (S) perçus par l'autre œil, où ladite mesure comprend les étapes consistant à :
- fournir une image statique, sur ledit moniteur (12), d'un index supérieur (11a) et d'un index inférieur (11b), alignés selon ledit plan médian transversal vertical ;
- fournir au moins une image, sur ledit moniteur (12) d'un stimulus (S) caractéristique de la mesure ;
- lorsque ladite image du stimulus (S) au minimum est fournie sur ledit moniteur (12), capturer une image (F) du visage du sujet examiné, au moyen desdits moyens de caméra vidéo frontale et afficher cette image sur le même moniteur (12), en arrière-plan par rapport à ladite image du stimulus (S) avec un contraste suffisant pour percevoir ladite image (F) du visage en arrière-plan,
où,
pour obtenir un positionnement correct de la tablette numérique autostéréoscopique (10), ladite tablette numérique autostéréoscopique (10) est orientée et disposée en positionnant lesdits indices (11a, 11b) l'un juste au-dessus du stimulus (S) et l'autre juste en dessous du stimulus (S), de sorte que l'indice supérieur (11a) soit positionné sur la partie centrale et supérieure du front du sujet examiné et que l'indice inférieur (11b) soit positionné sur le bout du nez du même sujet.

2. Procédé selon la revendication 1, **caractérisé par le fait que** ladite tablette numérique autostéréoscopique (10) est tenue par les mains du sujet examiné et est orientée et disposée, en positionnant lesdits indices (11a, 11b), l'un juste au-dessus du stimulus (S) et l'autre juste en dessous du stimulus (S), de sorte que l'indice supérieur (11a) soit positionné sur la partie centrale et supérieure du front du sujet examiné et que ladite extrémité proximale de l'indice inférieur (11b) soit positionnée sur le bout du nez du même sujet.

3. Procédé selon la revendication 1, **caractérisé par le fait que** ladite tablette numérique autostéréoscopique (10) est soutenue de manière stable au moyen d'un support pivotant, et **par le fait que** ledit support pivotant portant ladite tablette numérique autostéréoscopique (10) est orienté par les mains du sujet examiné et disposé, en positionnant lesdits indices (11a, 11b), l'un juste au-dessus du stimulus (S) et l'autre juste en dessous du stimulus (S), de sorte que l'indice supérieur (11a) soit positionné sur la partie centrale et supérieure du front du sujet examiné et que ladite extrémité proximale de l'indice inférieur (11b) soit positionnée sur le bout du nez du même sujet.

4. Procédé selon la revendication 1, **caractérisé par le fait que** ladite tablette numérique autostéréoscopique (10) est soutenue de manière stable au moyen d'un support pivotant, et **par le fait que** ledit support pivotant portant ladite tablette numérique autostéréoscopique est orienté par les mains d'un opérateur différent du sujet examiné et disposé, en positionnant lesdits indices (11a, 11b), l'un juste au-dessus du stimulus (S) et l'autre juste en dessous du stimulus (S), de sorte que l'indice supérieur (11a) soit positionné sur la partie centrale et supérieure du front du sujet examiné et que ladite extrémité proximale de l'indice inférieur (11b) soit positionnée sur le bout du nez du même sujet.

5. Procédé selon une ou plusieurs des revendications précédentes, **caractérisé par le fait que** ladite tablette numérique autostéréoscopique (10) est associée à une autre tablette numérique, sur laquelle est installé un logiciel dédié, qui est reliée de manière opérationnelle à ladite tablette numérique autostéréoscopique (10) au moyen d'un réseau de transmission de signaux, et sur le moniteur de laquelle sont reproduits, en temps réel par rapport à ladite tablette numérique autostéréoscopique (10) :
- ladite image statique, reproduite sur ledit moniteur de la tablette informatique autostéréoscopique (10), de deux index (11a, 11b), l'un supérieur (11a) et l'autre inférieur (11b), mutuellement alignés selon un plan médian transversal vertical du même moniteur (12) contenant l'axe optique desdits moyens de caméra vidéo ;
- ladite une image au minimum, reproduite sur ledit moniteur de la tablette numérique autostéréoscopique, d'un stimulus (S) caractéristique de la mesure ;
- ladite image (F) du visage du sujet examiné, prise au moyen desdits moyens de caméra vidéo frontale de la tablette numérique autostéréoscopique (10) et affichée sur le moniteur (12) de celle-ci, en arrière-plan par rapport à ladite image du stimulus (S) caractéristique de la mesure et présentée sur le moniteur (12) de ladite tablette numérique autostéréoscopique (10) avec un contraste juste suffisant pour percevoir ladite image (F) en arrière-plan.

6. Procédé selon la revendication 5, **caractérisé par le fait qu'**il comprend une étape de commande sélective de l'affichage, sur le moniteur (12) de ladite tablette numérique autostéréoscopique (10), de ladite image statique de deux index (11a, 11b), de ladite une image d'un stimulus (S) au minimum et/ou de ladite image (F) du visage du sujet examiné, par des moyens de commande à distance dudit affichage prévus dans ladite autre tablette numérique, de sorte qu'une, aucune ou une multitude desdites mêmes images sont visibles sur le moniteur (12) de ladite tablette numérique autostéréoscopique (10).

7. Procédé selon une ou plusieurs des revendications précédentes, **caractérisé par le fait que** ladite image (F) affichée en arrière-plan par rapport à ladite image du stimulus (S) caractéristique de la mesure, sur le moniteur (12) de ladite tablette numérique autostéréoscopique (10), est commandée à l'aide de moyens temporisés qui provoquent sa disparition après un intervalle de temps prédéterminé.

8. Procédé selon une ou plusieurs des revendications précédentes, **caractérisé par le fait que** ladite image dudit stimulus (S) au minimum est reproduite sur ledit moniteur (12) de ladite tablette numérique autostéréoscopique (10) dans une case à l'extérieur de laquelle le reste dudit moniteur (12) est gris ou rempli de caractères dans le but de créer une confusion.

9. Tablette numérique autostéréoscopique (10) configurée pour effectuer des mesures de disparité de fixation chez un sujet par observation haploscopique de stimuli, selon une ou plusieurs des revendications précédentes, dans laquelle ladite tablette numérique comprend :
- des moyens de caméra vidéo frontale pour capturer des images du visage du sujet, lesdits moyens de caméra vidéo frontale définissant un axe optique,
- un moniteur (12) ayant au moins 1920 x 1200 pixels avec une résolution d'au moins 100 pixels par mm², le moniteur définissant un plan médian transversal vertical contenant ledit axe optique,
- des moyens de reproduction d'images pour reproduire des images capturées par lesdits moyens de caméra vidéo sur ledit moniteur, et
- un logiciel dédié ;
- ledit moniteur étant configuré pour afficher :
- une image statique d'un index supérieur (11a) et d'un index inférieur (11b), mutuellement alignés selon ledit plan médian transversal vertical ;
- au moins une image d'un stimulus (S) caractéristique de la mesure ;
- une image (F) du visage du sujet, capturée par lesdits moyens de caméra vidéo frontale en arrière-plan par rapport à ladite image du stimulus (S) avec un contraste juste suffisant pour percevoir ladite image (F) du visage en arrière-plan,
de sorte que, pour obtenir un positionnement correct de la tablette numérique autostéréoscopique (10), ladite tablette numérique autostéréoscopique (10) peut être orientée et disposée en positionnant lesdits index (11a, 11b), l'un juste au-dessus du stimulus (S) et l'autre juste en dessous du stimulus (S), de sorte que l'index supérieur (11a) est positionné sur la partie centrale et supérieure du front du sujet examiné et que l'index inférieur (11b) est positionné sur le bout du nez du même sujet.

10. Tablette numérique autostéréoscopique selon la revendication 9, **caractérisée par le fait qu'**un moins une image dudit stimulus (S) occupe une case dudit moniteur de ladite tablette numérique autostéréoscopique (10) à l'extérieur de laquelle la partie restante dudit moniteur (12) est grise ou remplie de caractères dans le but de créer une confusion.

11. Tablette numérique autostéréoscopique selon la revendication 9 et/ou 10, **caractérisée par le fait que** lesdits deux index (11a, 11b), un supérieur (11a) et l'autre inférieur (11b) sont pointus, avec des points se faisant face, et mutuellement alignés selon un plan médian transversal vertical du même moniteur (12) contenant l'axe optique desdits moyens de caméra vidéo.

12. Tablette numérique autostéréoscopique selon la revendication 9, **caractérisée par le fait que** ladite tablette numérique autostéréoscopique (10) est soutenue de manière stable par un support pivotant.

13. Tablette numérique autostéréoscopique selon une ou plusieurs des revendications de 9 à 12, **caractérisée par le fait que** ladite tablette numérique autostéréoscopique (10), sur laquelle un logiciel dédié est installé, est connectée de manière opérationnelle à une autre tablette numérique, au moyen d'un réseau de transmission de signaux, et **par le fait que** sur le moniteur de ladite autre tablette numérique sont reproduits, en temps réel par rapport à ladite tablette numérique autostéréoscopique :
- ladite image statique, reproduite sur ledit moniteur (12) de la tablette numérique autostéréoscopique (10), de deux index (11a, 11b), l'un supérieur (11a) et l'autre inférieur (11b), mutuellement alignés selon un plan médian transversal vertical du même moniteur (12) contenant l'axe optique desdits moyens de caméra vidéo ;
- ladite image au minimum, reproduite sur ledit moniteur (12) de la tablette numérique autostéréoscopique (10), d'un stimulus (S) caractéristique de la mesure ;
- ladite image (F) du visage du sujet examiné, capturée au moyen desdits moyens de caméra vidéo frontale de la tablette numérique autostéréoscopique (10) et affichée sur le moniteur (12) de celle-ci, en arrière-plan par rapport à ladite image du stimulus (S) caractéristique de la mesure et présentée sur le moniteur (12) de ladite tablette numérique autostéréoscopique (10) avec un contraste juste suffisant pour percevoir ladite image (F) en arrière-plan.

14. Tablette numérique autostéréoscopique selon la revendication 13, **caractérisée par le fait qu'**elle comprend des moyens de commande à distance, prévus dans ladite autre tablette numérique, de l'affichage, sur le moniteur (12) de ladite tablette numérique autostéréoscopique (10), de ladite image statique de deux index (11a, 11b), de ladite image d'un stimulus (S) au minimum et/ou de ladite image (F) du visage du sujet examiné, qui permettent de rendre visible sur le moniteur (12) de ladite tablette numérique autostéréoscopique (10) une, aucune ou une multitude desdites mêmes images.
